# EUROPEAN PATENT SPECIFICATION

(11) **EP 3 730 073 B1**
(45) Date of publication and mention of the grant of the patent: **30.10.2024**
(21) Application number: 20170564.7
(22) Date of filing: 21.04.2020
(51) Int. Cl.: A61B 17/115, A61B 17/00

(54) **SURGICAL DEVICES INCLUDING TROCAR LOCK AND TROCAR CONNECTION INDICATOR**
CHIRURGISCHE VORRICHTUNGEN MIT TROKARVERSCHLUSS UND TROKARVERBINDUNGSINDIKATOR
DISPOSITIFS CHIRURGICAUX COMPRENANT UN VERROUILLAGE DE TROCART ET INDICATEUR DE CONNEXION DE TROCART

(30) Priority: 22.04.2019 US 201962836950 P; 22.04.2019 US 201962836933 P; 22.04.2019 US 201962836918 P; 25.03.2020 US 202016829185
(43) Date of publication of application: 28.10.2020
(73) Proprietor: Covidien LP, Mansfield, MA 02048 (US)
(72) Inventor: EISINGER, Joseph, Northford, Connecticut 06472 (US); CABRERA, Ramiro, Cheshire, Connecticut 06410 (US)
(74) Representative: Maschio & Soames IP Ltd

(56) References cited:
- EP-A1- 3 078 335
- EP-A1- 3 146 905
- EP-A1- 3 192 462
- EP-A2- 3 666 198
- US-A1- 2017 333 077
- US-A1- 2018 280 024
- KRAFT TOOL CO.: "Kraft tool Produktblad 2018", 11 July 2018 (2018-07-11), XP055730489, Retrieved from the Internet <URL:https://www.slideshare.net/BetongakutenAB/kraft-tool-produktblad-2018> [retrieved on 20200914]

## Description

### CROSS-REFERENCE TO RELATED APPLICATIONS

The present application claims the benefit of and priority to U.S. Provisional Patent Application Serial No. 62/836,950, filed on April 22, 2019.

The present application claims the benefit of and priority to U.S. Provisional Patent Application Serial No. 62/836,933, filed on April 22, 2019.

The present application claims the benefit of and priority to U.S. Provisional Patent Application Serial No. 62/836,918, filed on April 22, 2019.

The present application is also a Continuation-in-Part Application which claims the benefit of and priority to U.S. Patent Application Serial No. 15/972,606, filed on May 7, 2018.

### BACKGROUND

### 1. Technical Field

The present disclosure relates to powered surgical devices. More specifically, the present disclosure relates to reusable handheld electromechanical surgical devices including trocar locks, and trocar connection indicators.

### 2. Background of Related Art

Circular stapling instruments for performing surgical procedures such as anastomoses, hemorrhoidectomies, and mucosectomies are well known. These devices include an anvil assembly having a center rod and an anvil head supported on the center rod. The center rod of the anvil assembly is attachable to a trocar of the circular stapling instrument which enable linear translation of the anvil assembly. Typically, during a surgical procedure, the tool assembly of the circular stapling instrument is inserted into a tubular section or sections of tissue to join the tissue sections or remove diseased or damaged tissue from within the tissue section. Following a surgical procedure, it is desirable to reprocess the circular stapling instrument in order to minimize overall costs of the surgical procedures. The reprocessing typically requires cleaning of the circular stapling instrument via autoclaving and the like. In order to improve the efficiency of the reprocessing, removability of the trocar is desirable.

Accordingly, in view thereof, it is desirable to provide locks and indicators which alert the end user (e.g., doctor, nurse, clinician, etc.) that a trocar is properly attached to the underlying circular stapling instrument.

Document US2018280024 A1 relates to circular anastomosis instruments, particularly, with instruments intended to be reused and sterilized in whole or in part. In one embodiment, a surgical circular fastener apparatus includes an elongated body defining a longitudinal axis and having proximal and distal ends, a fastener cartridge disposed adjacent the distal end of the body, an anvil retainer or trocar releasably mounted relative to the fastener cartridge and a manually operably release configured to move between ` a first position corresponding to a secured condition of the anvil retainer relative to the fastener cartridge and a second position corresponding to a release condition of the anvil retainer relative to the fastener cartridge. The anvil retainer or trocar may be couplable to an anvil assembly. The elongated body includes a release housing with the manually operable release being mounted for movement relative to the release housing. The release housing may include at least one lock. The at least one lock may be operatively coupled to the manually operable release and configured to move between a locked position in secured engagement with the anvil retainer to prevent removal of the anvil retainer relative to the fastener cartridge upon movement of the manually operable release to the first position, and an unlocked position released from the anvil retainer to permit removal or mounting of the anvil retainer relative to the fastener cartridge upon movement of the manually operable release to the second position.

### SUMMARY

The invention is defined by appended claim 1.

### BRIEF DESCRIPTION OF THE DRAWINGS

Embodiments of the present disclosure are described herein with reference to the accompanying drawings, wherein:
FIG. 1 is a perspective view of a handheld surgical device including a handle assembly, an adapter assembly, and a reload in accordance with an embodiment of the present disclosure;
FIG. 2 is a perspective view, with parts separated, of the handle assembly of FIG. 1;
FIG. 3 is a perspective view, with parts separated, of a motor assembly and a control assembly of a power handle of the handle assembly of FIG. 2;
FIG. 4 is a perspective view of the adapter assembly without the reload secured to a distal end thereof;
FIG. 5 is a cross-sectional view of the adapter assembly of FIG. 4, as taken through section line 5-5 of FIG. 4;
FIG. 6 is a cross-sectional view of the adapter assembly of FIG. 4, as taken through section line 6-6 of FIG. 4;
FIG. 7 is a perspective view of the adapter assembly, shown partially in phantom, illustrating a first force/rotation transmitting/converting assembly thereof;
FIG. 8 is a perspective view of the first force/rotation transmitting/converting assembly of FIG. 7;
FIG. 9 is a perspective view, with parts separated, of a trocar assembly of the first force/rotation transmitting/converting assembly of FIG. 7;
FIG. 10 is a perspective view, of a distal end portion of the first force/rotation transmitting/converting assembly of FIG. 7, illustrating a support block thereof, and a trocar lock assembly associated therewith;
FIG. 11 is a perspective view, of a distal end portion of the first force/rotation transmitting/converting assembly of FIG. 7, with the support block thereof shown in phantom;
FIG. 12 is a cross-sectional view as taken through 12-12 of FIG. 10;
FIG. 13 is a cross-sectional view as taken through 13-13 of FIG. 10;
FIG. 14 is a cross-sectional view as taken through 14-14 of FIG. 13;
FIG. 15 is an enlarged view of the indicated area of detail of FIG. 4;
FIG. 16 is a cross-sectional view as taken through 16-16 of FIG. 15;
FIG. 17 is a cross-sectional view as taken through 17-17 of FIG. 15;
FIG. 18 is a perspective view, of a distal end portion of the first force/rotation transmitting/converting assembly of FIG. 7, illustrating the trocar lock assembly associated therewith in a depressed condition;
FIG. 19 is a perspective view, of a distal end portion of the first force/rotation transmitting/converting assembly of FIG. 7, illustrating the trocar lock assembly associated therewith in an undepressed condition;
FIG. 20 is a perspective view of a distal end of an adapter assembly, without the reload secured to a distal end thereof, illustrating a trocar lock assembly according to an embodiment of the invention;
FIG. 21 is a top, plan view of the trocar lock assembly of FIG. 20, with an outer tube of the adapter assembly removed;
FIG. 22 is a top, plan view of the trocar lock assembly of FIG. 20, with an outer tube and a support block of the adapter assembly removed, illustrating the trocar lock assembly in a second locked position;
FIG. 23 is an enlarged view of the indicated area of detail of FIG. 22, illustrating the trocar lock assembly in a first unlocked position;
FIG. 24 is a perspective view of the adapter assembly of FIG. 4, shown partially in phantom, illustrating a second force/rotation transmitting/converting assembly thereof; and
FIG. 25 is a perspective view of the adapter assembly of FIG. 4, shown partially in phantom, illustrating a third force/rotation transmitting/converting assembly thereof.

### DETAILED DESCRIPTION OF EMBODIMENTS

Embodiments of the present disclosure are now described in detail with reference to the drawings, in which like reference numerals designate identical or corresponding elements in each of the several views. As used herein the term "proximal" refers to a portion of a surgical device, or component thereof, closer to the user, and the term "distal" refers to a portion of the surgical device, or component thereof, farther from the user.

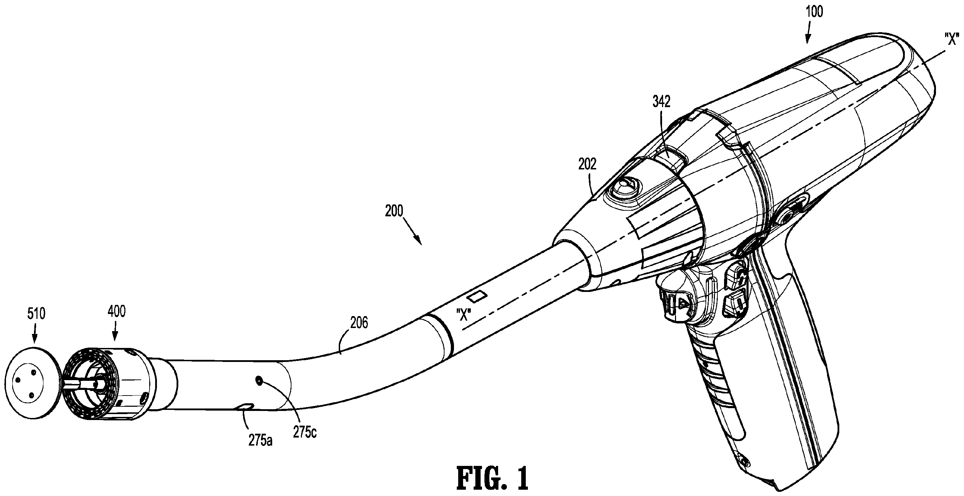

Turning now to FIG. 1, a surgical device 10, in accordance with an embodiment of the present disclosure, is in the form of a powered handheld electromechanical instrument. The surgical device includes a handle assembly 100, an adapter assembly 200, a reload 400, and an anvil assembly 510. The handle assembly 100 is configured for selective connection with the adapter assembly 200 and, in turn, the adapter assembly 200 is configured for selective connection with the reload 400.

The handle assembly 100, the adapter assembly 200, and the reload 400 will only further be described to the extent necessary to disclose aspects of the present disclosure. For a detailed description of the structure and function of exemplary handle assemblies, adapter assemblies, and reloads, reference may be made to commonly owned U.S. Patent Appl. Pub. No. 2016/0310134 and U.S. Patent Appl. Serial No. 15/972,606.

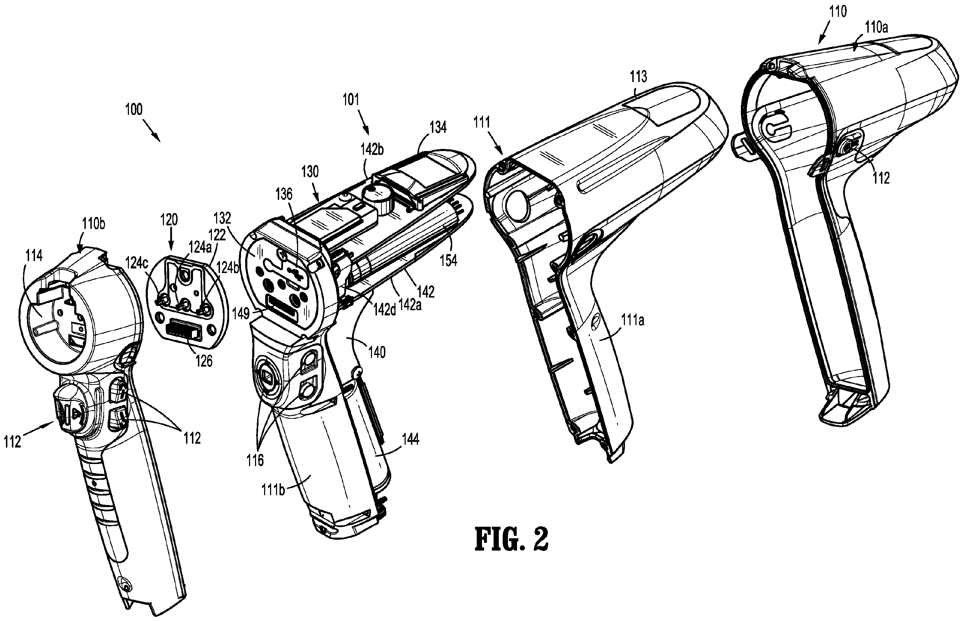

With reference now to FIG. 2, the handle assembly 100 includes a power handle 101 and an outer or shell housing 110 configured to selectively receive and encase the power handle 101. The shell housing 110 includes a proximal half-section 110a and a distal half-section 110b that are couplable together. The shell housing 110 includes a plurality of actuators 112 (e.g., finger-actuated control buttons, knobs, toggles, slides, interfaces, and the like) for activating various functions of the surgical device 10 (FIG. 1) upon application of a respective force thereto.

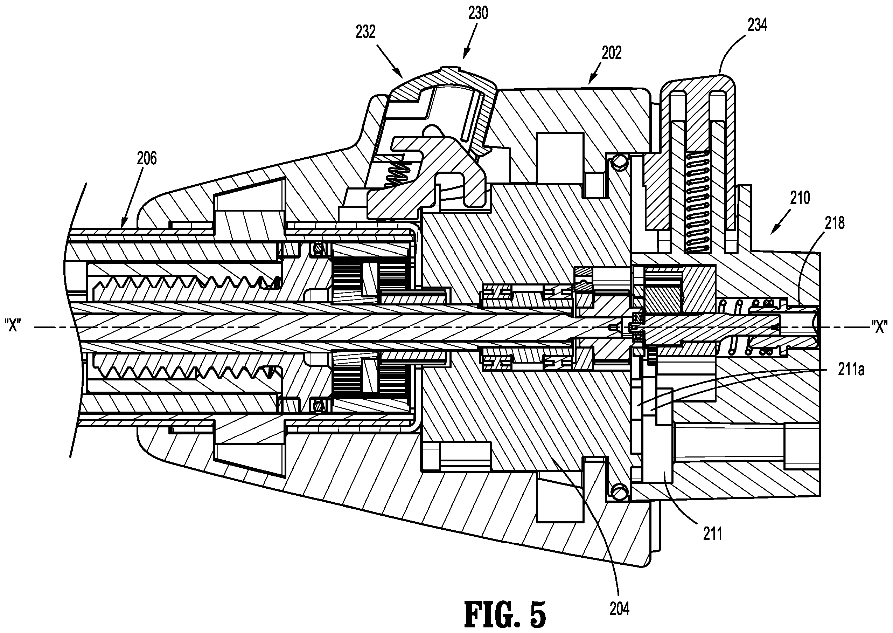

The distal half-section 110b of the shell housing 110 defines a connecting portion 114 (e.g., a recess) configured to accept or receive a corresponding drive coupling assembly 210 (FIG. 5) of the adapter assembly 200. A sterile barrier plate assembly 120 is selectively supported in the distal half-section 110b of the shell housing 110 behind the connection portion 114. The plate assembly 120 includes a plate 122 rotatably supporting three coupling shafts 124a, 124b, 124c, and having an electrical connector 126 supported thereon. The electrical connector 126 includes a chip and defines a plurality of contact paths each including an electrical conduit for extending an electrical connection across the plate 122. When the plate assembly 120 is disposed within the shell housing 110, distal ends of the coupling shafts 124a, 124b, 124c and the electrical connector 126 are disposed or situated within the connecting portion 114 of the shell housing 110 to electrically and/or mechanically engage respective corresponding features of the adapter assembly 200, as will be described in greater detail below.

The power handle 101 has an inner handle housing 111 including a proximal half section 111a and a distal half section 111b that are coupled together to house a power-pack core assembly 130 therein. The power-pack core assembly 130 is configured to control the various operations of the handle assembly 100 and thus, the surgical device 10.

The distal half section 111b of the inner handle housing 111 is configured and adapted to support a control plate 132 of the power-pack core assembly 130 such that the control plate 132 abuts the plate assembly 120 of the shell housing 110 when the power handle 101 is disposed within the shell housing 110. The distal half section 111b of the inner handle housing 111 also supports a plurality of actuator interfaces 116 that are in operative registration with the respective actuators 112 of the shell housing 110.

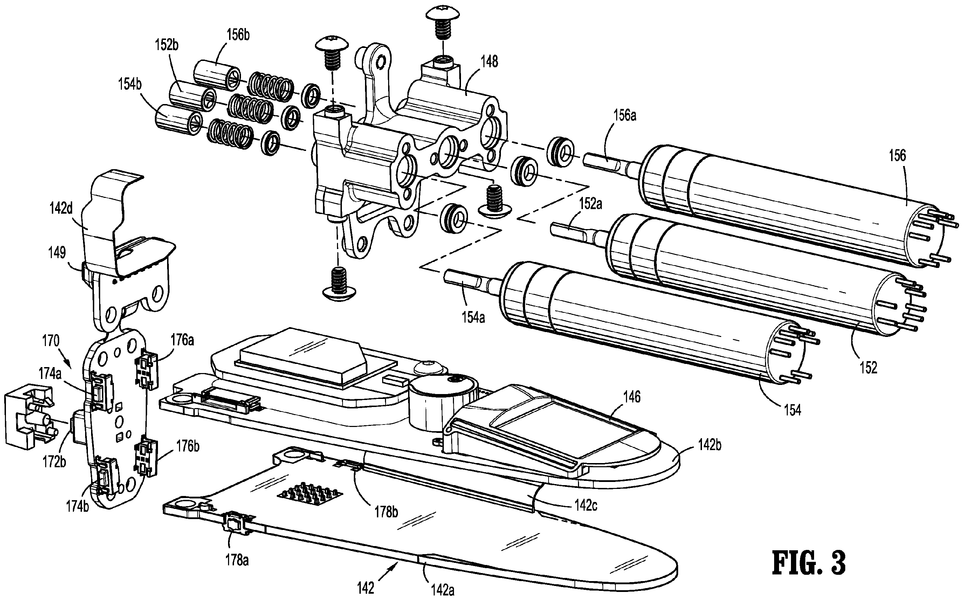

As shown in FIGS. 2 and 3, the power-pack core assembly 130 includes a battery circuit 140, a controller circuit board 142, and a rechargeable battery 144 configured to supply power to any of the electrical components of the handle assembly 100. The controller circuit board 142 includes a motor controller circuit board 142a, a main controller circuit board 142b, and a first ribbon cable 142c interconnecting the motor controller circuit board 142a and the main controller circuit board 142b. A display screen 134 is supported on the main controller circuit board 142b and visible through a clear or transparent window 113 provided in the proximal half-section 111a of the inner handle housing 111. A USB connector 136 (or other data connector) is also supported on the main controller circuit board 142b and is accessible through the control plate 132 of the power-pack core assembly 130.

The power-pack core assembly 130 further includes a first motor 152, a second motor 154, and a third motor 156 disposed between the motor controller circuit board 142a and the main controller circuit board 142b. Each of the first, second, and third motors 152, 154, 156 is electrically connected to the controller circuit board 142 and the battery 144, and controlled by a respective motor controller disposed on the motor controller circuit board 142a which, in turn, is coupled to a respective main controller disposed on the main controller circuit board 142b.

Each of the first, second, and third motors 152, 154, 156 is supported on a motor bracket 148 such that respective motor shaft 152a, 154a, 156a extending from the first, second, and third motors 152, 154, 156 are rotatably disposed within respective apertures of the motor bracket 148. The motor bracket 148 rotatably supports three rotatable drive connector sleeves 152b, 154b, 156b that are keyed to the respective motor shafts 152a, 154a, 156a of the first, second, and third motors 152, 154, 156. The drive connector sleeves 152b, 154b, 156b non-rotatably receive proximal ends of the respective coupling shafts 124a, 124b, 124c of the plate assembly 120 of the shell housing 110, when the power handle 101 is disposed within the shell housing 10, and are each spring biased away from the respective motors 152, 154, 156.

The motor bracket 148 also supports an electrical receptacle 149. The electrical receptacle 149 is in electrical connection with the main controller circuit board 142b by a second ribbon cable 142d. The electrical receptacle 149 defines a plurality of electrical slots for receiving respective electrical contacts or blades extending from the pass-through connector 126 of the plate assembly 120 of the shell housing 110.

Rotation of the motor shafts 152a, 154a, 156a by the respective first, second, and third motors 152, 154, 156 function to drive shafts and/or gear components of the adapter assembly 200 in order to perform the various operations of the handle assembly 100, as will be described in greater detail below.

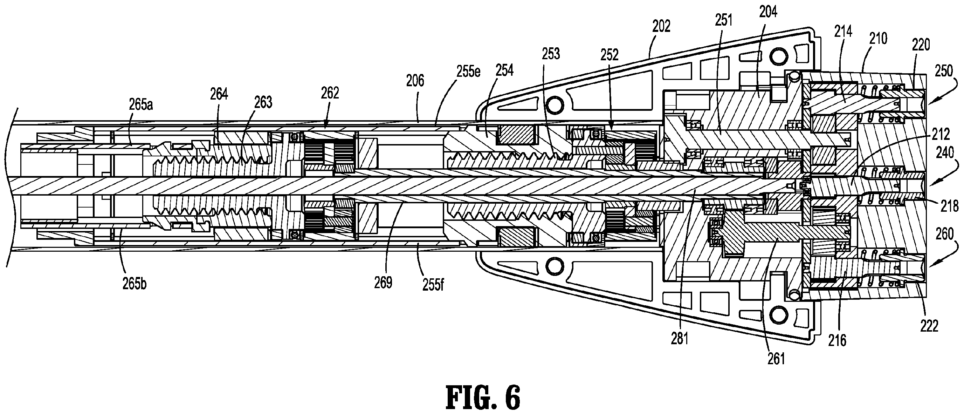

In use, when the adapter assembly 200 is mated to the handle assembly 100, each of the coupling shafts 124a, 124b, 124c of the handle assembly 100 couples with a corresponding rotatable connector sleeve 218, 222, 220 (FIG. 6) of the adapter assembly 200. In this regard, the interface between corresponding coupling shafts 124a, 124b, 124c and connector sleeves 218, 222, 220 are keyed such that rotation of each of the coupling shafts 124a, 124b, 124c of the handle assembly 100 causes a corresponding rotation of the corresponding connector sleeve 218, 222, 220 of the adapter assembly 200.

The coupling shafts 124a, 124b, 124c of handle assembly 100 are configured to be independently rotated by the respective motor 152, 154, 156 such that rotational force(s) are selectively transferred from the motors 152, 154, 156 of the handle assembly 100 to the adapter assembly 200. The selective rotation of the coupling shaft(s) 124a, 124b, 124c of the handle assembly 100 allows the handle assembly 100 to selectively actuate different functions of the reload 400.

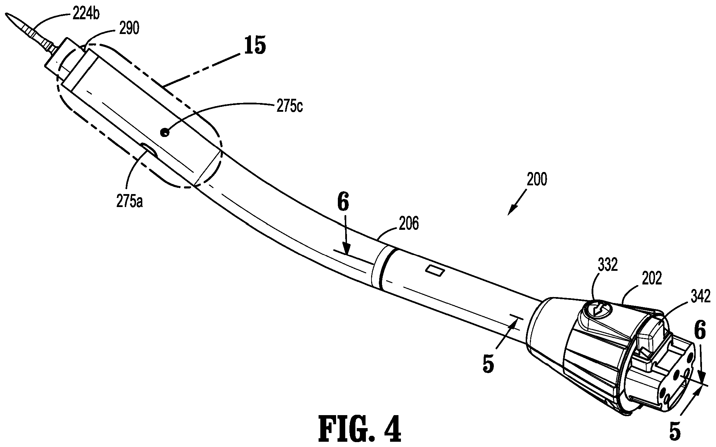

Turning now to FIG. 4, the adapter assembly 200 is configured to convert a rotation of the coupling shaft(s) 124a, 124b, 124c (FIG. 2) of the handle assembly 100 into axial translation useful for effecting various functions of the surgical device 10 (FIG. 1). The adapter assembly 200 includes an adapter or knob housing 202 and an outer tube 206 extending from a distal end of the knob housing 202. The knob housing 202 and the outer tube 206 are configured and dimensioned to house and support the components of the adapter assembly 200. The knob housing 202 includes a drive coupling assembly 210 which is configured and adapted to connect to the connecting portion 114 (FIG. 2) of the shell housing 110 of the handle assembly 100. The outer tube 206 includes a connector sleeve 290 fixedly supported at a distal end thereof. The connector sleeve 290 is configured to selectively secure the reload 400 (FIG. 1) to the adapter assembly 200.

As shown in FIGS. 4 and 5, the adapter assembly 200 includes a rotation assembly 230 configured to enable rotation of the adapter assembly 200 relative to the handle assembly 100. Specifically, the knob housing 202 and the outer tube 206 of the adapter assembly 200 are rotatable relative to the drive coupling assembly 210 of the adapter assembly 200. The rotation assembly 230 includes a lock button 232 operably supported on the knob housing 202 and configured for actuating the rotation assembly 230. When rotation assembly 230 is in an unlocked configuration, the knob housing 202 and the outer tube 206 are rotatable along a longitudinal axis "X" of the adapter assembly 200 relative to the drive coupling assembly 210. When rotation assembly 230 is in a locked configuration, the knob housing 202 and the outer tube 206 are rotationally secured relative to the drive coupling assembly 210.

The adapter assembly 200 further includes an attachment/detachment button 234 supported on the drive coupling assembly 210 of the adapter assembly 200. In use, when the adapter assembly 200 is connected to the shell housing 110 of the handle assembly 100, the attachment/detachment button 234 secures and retains the adapter assembly 200 and the handle assembly 100 with one another. When the attachment/detachment button 234 is depressed or actuated, the adapter assembly 200 and the handle assembly 100 may be disconnected from each other.

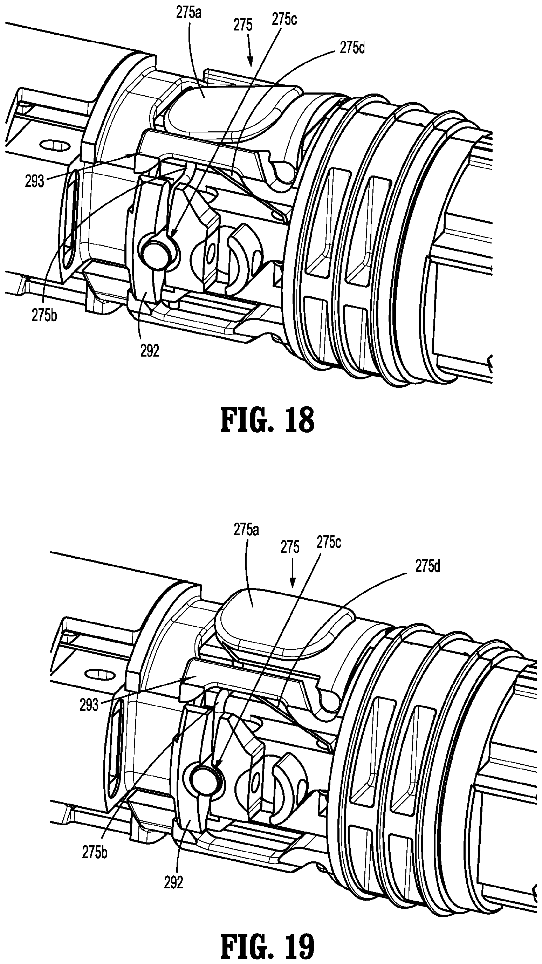

The adapter assembly 200 further includes a cavity 211 defined within the drive coupling assembly 210 that is configured to receive a pin connector assembly 320 (FIG. 18) of an electrical assembly 300 configured for establishing an electrical connection with and between the handle assembly 100, the adapter assembly 200, and the reload 400, as described in further detail below. The cavity 211 may include guiding ribs 211 configured to receive a printed circuit board 324 of the pin connector assembly 320.

As illustrated in FIG. 6, the drive coupling assembly 210 of the adapter assembly 200 rotatably supports first, second, and third connector sleeves 218, 220 and 222 therein, and an inner housing member 204 disposed within the knob housing 202 rotatably supports first, second, and third rotatable proximal drive shafts 212, 214, 216 therein. Each of the first, second, and third connector sleeves 218, 220, 222 is configured to mate with a respective coupling shaft 124a, 124c, 124b (FIG. 2) of the handle assembly 100. Each of the first, second, and third connector sleeves 218, 220, 222 is further configured to mate with a proximal end of the respective first, second, and third proximal drive shafts 212, 214, 216 of the adapter assembly 200 such that each of the first, second, and third proximal drive shafts 212, 214, 216 functions as a rotation receiving member to receive rotational forces from the respective coupling shafts 124a, 124c, 124b of the handle assembly 100.

The adapter assembly 200 includes first, second and third force/rotation transmitting/converting assemblies 240, 250, 260 disposed within the inner housing member 204 and the outer tube 206. Each of the force/rotation transmitting/converting assemblies 240, 250, 260 is configured and adapted to transmit or convert a rotation of the respective coupling shaft 124a, 124c, 124b of the handle assembly 100 into axial translation to effectuate operation of the reload 400 (FIG. 1), as will be described in greater detail below.

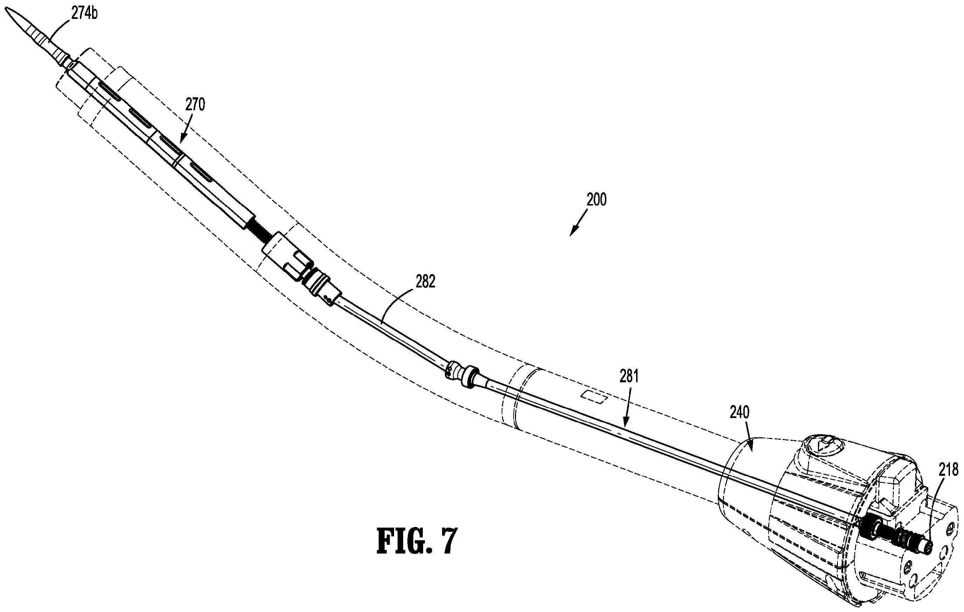

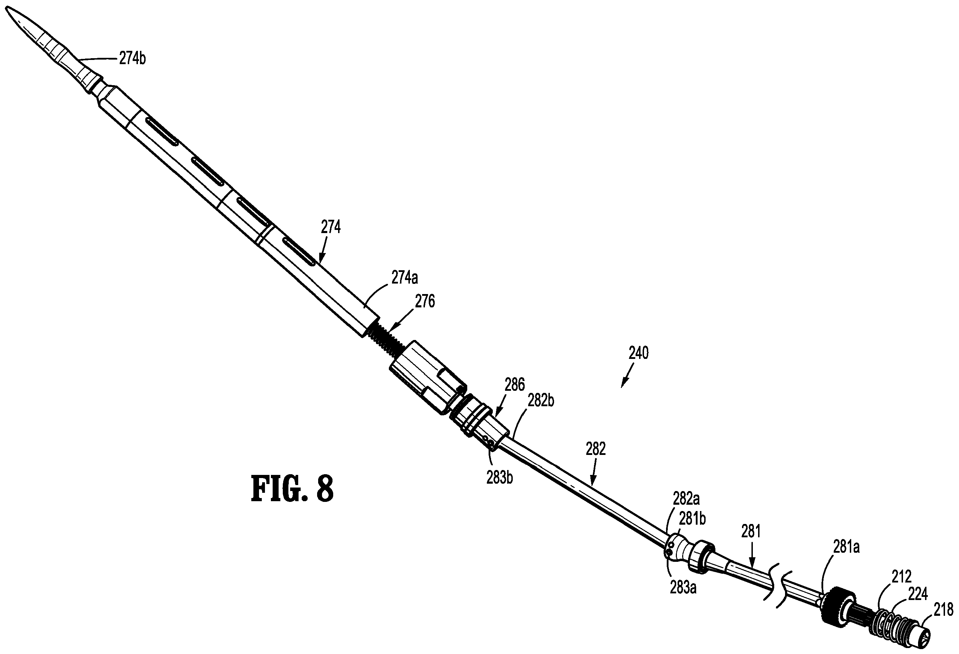

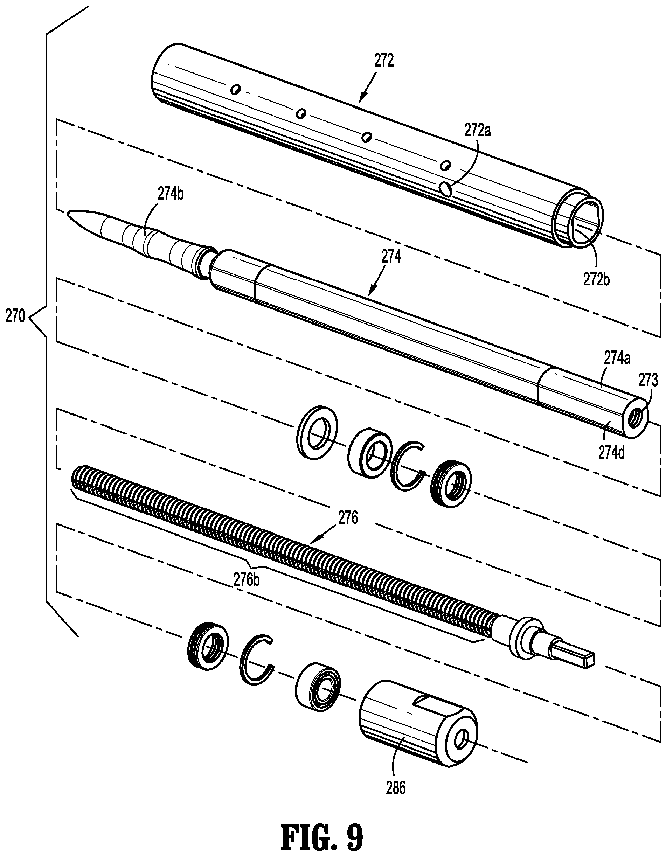

As shown in FIGS. 6 and 7, the first force/rotation transmitting/converting assembly 240 includes the first rotatable proximal drive shaft 212, as described above, a second rotatable proximal drive shaft 281, a rotatable distal drive shaft 282, and a coupling member 286. First force/rotation transmitting/converting assembly 240, as illustrated in FIGS. 7-19, further includes a trocar assembly 270 removably supported in a distal end of outer tube 206. Trocar assembly 270 includes a tubular outer housing 272, a trocar member 274 slidably disposed within tubular outer housing 272, and a drive screw 276 operably received within trocar member 274 for axially moving trocar member 274 relative to tubular housing 272. In particular, trocar member 274 includes a proximal end 274a having an inner threaded portion 273 which engages a threaded distal portion 276b of drive screw 276. Trocar member 274 further includes at least one longitudinally extending flat 274d formed in an outer surface thereof which mates with a corresponding flat 272b formed in tubular housing 272 thereby inhibiting rotation of trocar member 274 relative to tubular housing 272 as drive screw 276 is rotated. A distal end 274b of trocar member 274 is configured to selectively engage anvil assembly 510 (FIG. 1).

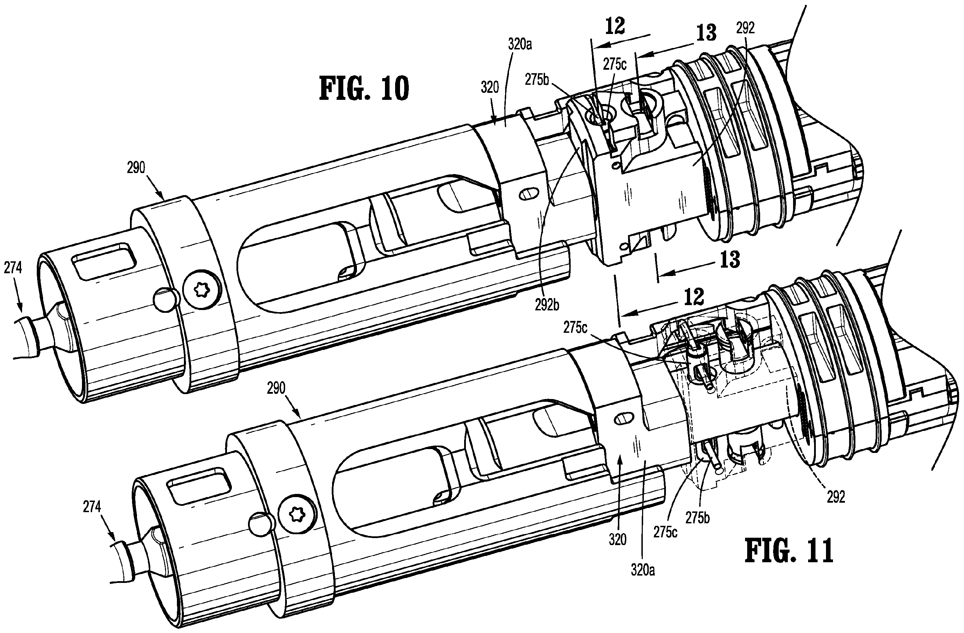

Tubular housing 272 of trocar assembly 270 is axially and rotationally fixed within outer tube 206 of adapter assembly 200. Tubular housing 272 defines a pair of radially opposed, and radially oriented openings 272a which are configured and dimensioned to cooperate with a pair of lock pins 275c of a trocar assembly release mechanism 275 (see FIGS. 10-14 and 17) of adapter assembly 200. With continued reference to FIGS. 10-19, adapter assembly 200 includes a support block 292 fixedly disposed within outer tube 206. The pair of lock pins 275c extend through support block 292 and into tubular housing 272 of trocar assembly 270 to connect trocar assembly 270 to adapter assembly 200.

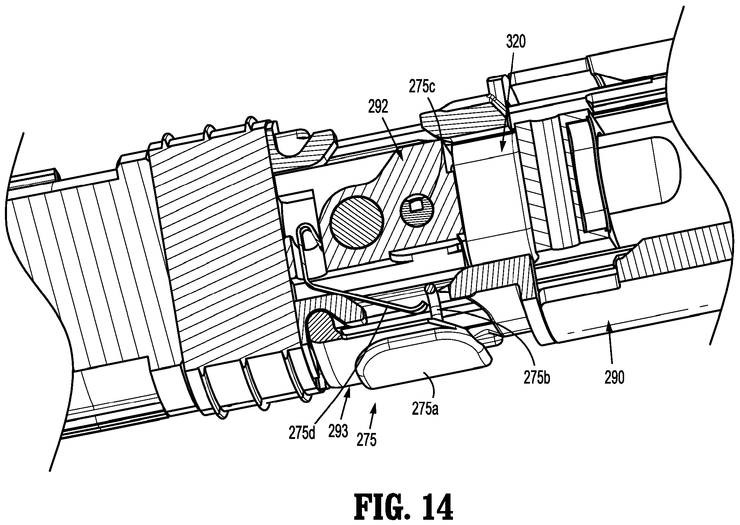

As illustrated in FIGS. 11-17, trocar assembly release mechanism 275 includes a release button 275a pivotally supported on a hinge guide 293 and in outer tube 206. Release button 275a is biased, via a spring 275d (see FIGS. 14, 18 and 19), to a locked/extended condition. Trocar assembly release mechanism 275 further includes a spring clip 275b having a backspan connected to release button 275a, and a pair of legs, extending from the backspan, that extend through support block 292 and transversely across trocar assembly 270. Each of the pair of legs of spring clip 275b extends through a respective lock pin 275c which is slidably disposed within a respective radial opening 272a of tubular housing 272 and radial opening 292a of support block 292. Each of the pair of legs of spring clip 275b defines a gooseneck along a length thereof such that a distal portion of each of the pair of legs of spring clip 275b is closer to one another as compared to a proximal portion of each of the pair of legs of spring clip 275b.

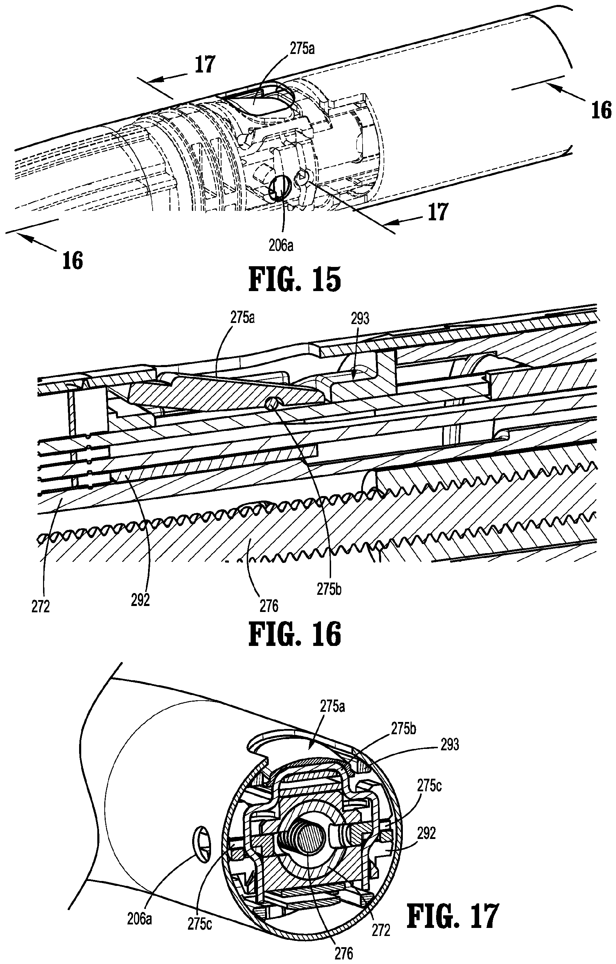

In use, when release button 275a is depressed (e.g., in a radially inward direction, FIGS. 15-18), release button 275a moves spring clip 275b transversely relative to trocar assembly 270. As spring clip 275b is moved transversely relative to trocar assembly 270, the pair of legs of spring clip 275b translate through the pair of lock pins 275c such that a gooseneck in each leg acts to cam and urge the pair of lock pins 275c radially outward. Specifically, the pair of lock pins 275c are traversed by the gooseneck portions the pair of legs of spring clip 275b, transitioning from the distal portions thereof to the proximal portions thereof. In so moving, each of the pair of lock pins 275c is urged radially outward by a distance sufficient that each of the pair of lock pins 275c clears respective opening 272a of tubular housing 272, and in an embodiment, project from outer tube 206 or are flush with an outer surface of outer tube 206. It is contemplated that outer tube 206 may include openings 206a (see FIGS. 15 and 17) formed therein which are in registration with each of the pair of lock pins 275c. With the pair of lock pins 275c free and clear of tubular housing 272, trocar assembly 270 may be axially withdrawn from within the distal end of outer tube 206 of adapter assembly 200, or may be inserted into the distal end of outer tube 206 of adapter assembly 200.

Projection of the pair of lock pins 275c, radially outward from outer tube 206 (or to be substantially flush with an outer surface of the outer tube 206), provides a visual indication to the end user that no trocar assembly 270 is inserted into the distal end of outer tube 206 of adapter assembly 200, or that trocar assembly 270 is not properly inserted into the distal end of outer tube 206 of adapter assembly 200. When trocar assembly 270 is properly inserted into the distal end of outer tube 206 of adapter assembly 200, the pair of lock pins 275c of trocar assembly release mechanism 275 are in registration with, and enter into, a respective opening 272a of tubular housing 272 of trocar assembly 270 (see FIG. 19), to thereby lock trocar assembly 270 within the distal end of outer tube 206 of adapter assembly 200, and to thereby provide a visual indication to the end user that trocar assembly 270 is properly inserted into the distal end of outer tube 206 of adapter assembly 200.

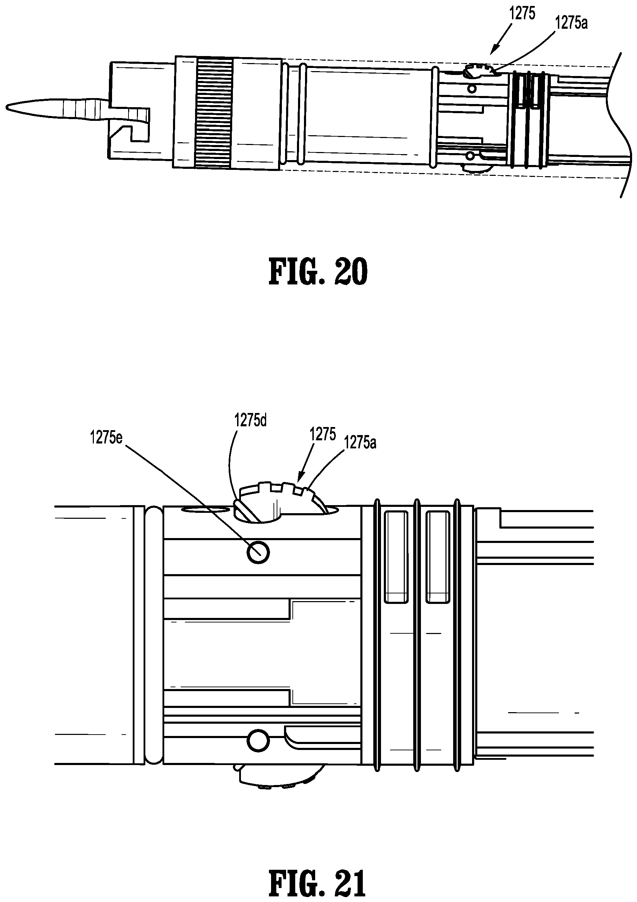

Turning now to FIGS. 20-23, disclosing an embodiment according to the invention, adapter assembly 200 includes a trocar assembly release mechanism 1275. As illustrated, trocar assembly release mechanism 1275 includes a pair of release buttons 1275a rotatably supported on a support block (not shown) and in outer tube 206, via a respective pivot pin 1275e. Each release button 1275a is biased, via a respective biasing member 1275d (e.g., leaf spring), to a locked condition.

Each release button 1275a is identical to one another, and thus, only one of the pair of release buttons 1275a will be described in detail herein. Release button 1275a is substantially semi-circular, extending approximately 180° about pivot pin 1275e. Release button 1275a defines a distal face or surface 1275a' against which a portion of biasing member 1275d engages to urge release button 1275a proximally, and a proximal face or surface projecting to a tail 1275a". An outer surface of release button 1275a may include finger gripping features (e.g., ribs, knurling, etc.) formed thereon.

Release button 1275a is movable between a first unlocked position and a second locked position. If or when a trocar assembly 270 is not properly inserted into the distal end of outer tube 206 of adapter assembly 200, the release button 1275a is rotated or urged radially outward from outer tube 206 by trocar assembly 270, to the first unlocked position, thereby providing a visual indication to the end user that trocar assembly 270 is not properly inserted into the distal end of outer tube 206 of adapter assembly 200. When a trocar assembly 270 is properly inserted into the distal end of outer tube 206 of adapter assembly 200, the release button 1275a is rotated or urged radially inward, to the second locked position, by biasing member 1275d rotating release button 1275a such that tail 1275a" thereof is received in a recess or depression formed in the outer surface of tubular housing 272 of trocar assembly 270 (e.g., similar to openings 272a of tubular housing 272). When release button 1275a is in the second locked position, a visual indication is provided to the end user that trocar assembly 270 is properly inserted and locked into the distal end of outer tube 206 of adapter assembly 200.

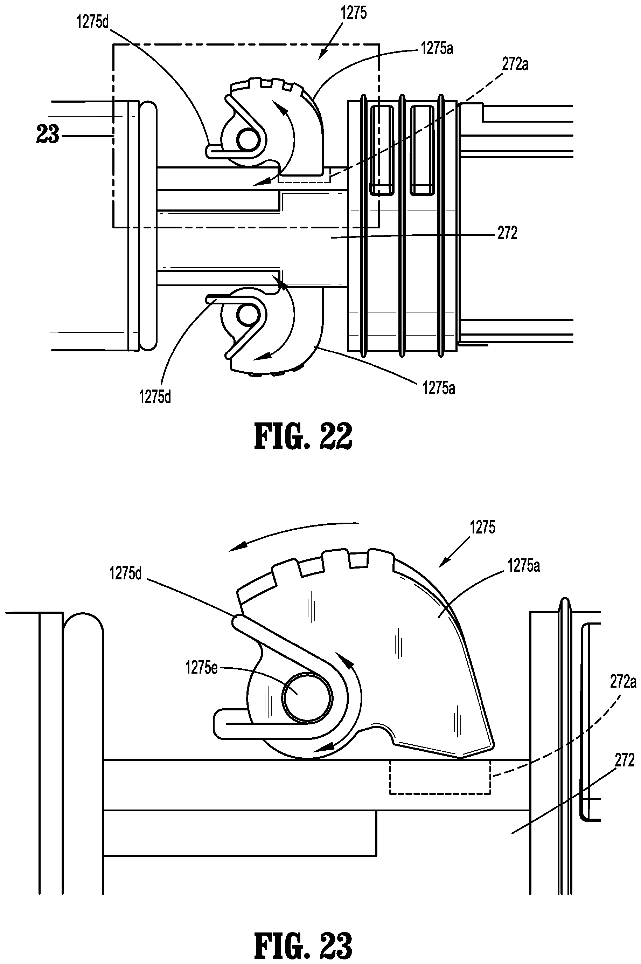

In an embodiment, with reference to FIG. 23, tail 1275a" includes a chamfered distal surface to facilitate receipt or passage of trocar assembly 270 during connection of trocar assembly 270 to outer tube 206, when release button 1275a is in the second locked position. Tail 1275a" further includes a substantially squared proximal surface to inhibit withdrawal or disconnection of trocar assembly 270 from outer tube 206 when release button 1275a is in the second locked position relative to tubular housing 272 of trocar assembly 270.

With reference to FIGS. 1-9, in operation, the first force/rotation transmitting/converting assembly 240 functions to advance/retract trocar member 274 of trocar assembly 270 of the adapter assembly 200, and to open/close the reload 400 (FIG. 1) when an anvil assembly 510 is connected to the trocar member 274. Specifically, as the first rotatable proximal drive shaft 212 is rotated, due to a rotation of the first connector sleeve 218, as a result of the rotation of the first coupling shaft 124a (FIG. 2) of the handle assembly 100, the second rotatable proximal drive shaft 281 is caused to be rotated. Rotation of the second rotatable proximal drive shaft 281 results in contemporaneous rotation of the rotatable distal drive shaft 282. Rotation of the rotatable distal drive shaft 282 causes contemporaneous rotation of the coupling member 286, which, in turn, causes contemporaneous rotation of the drive screw 276 of the trocar assembly 270. As the drive screw 276 is rotated within and relative to the trocar member 274, engagement between the trocar member 274 and the drive screw 276 (e.g., threaded engagement) causes axial translation of the trocar member 274 within the tubular housing 272 of the trocar assembly 270. Specifically, rotation of the drive screw 276 in a first direction causes axial translation of the trocar member 274 in a first direction (e.g., extension or advancement of the trocar assembly 270), and rotation of the drive screw 276 in a second direction causes axial translation of the trocar member 274 in a second direction (e.g., retraction of the trocar assembly 270). When the anvil assembly 510 is connected to the trocar member 274, the axial translation of the trocar member 274 in the first direction results in an opening of the reload 400, and the axial translation of the trocar member 274 in the second direction results in a closing of the reload 400.

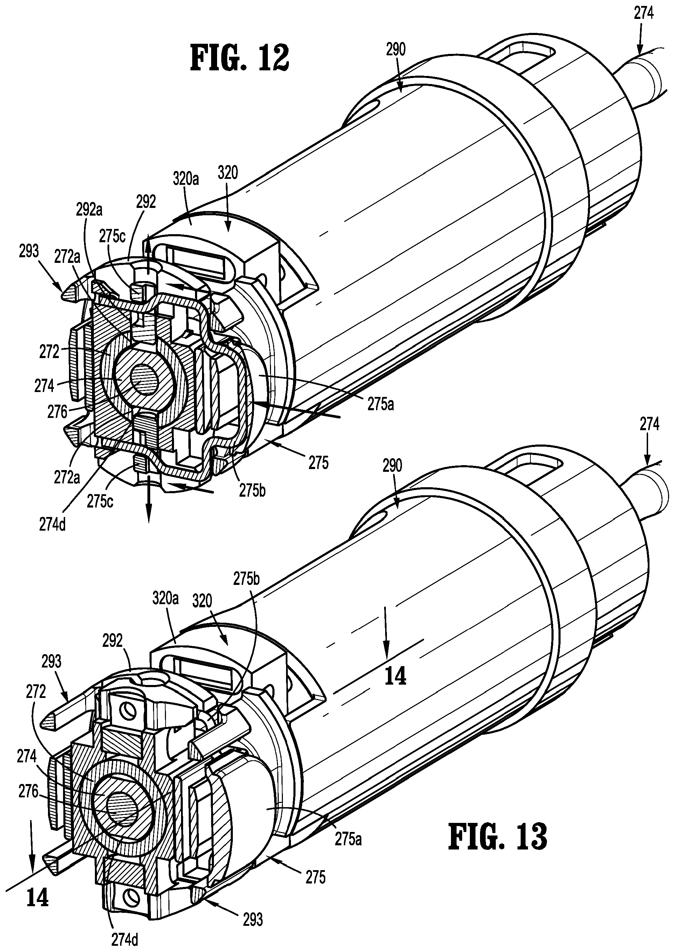

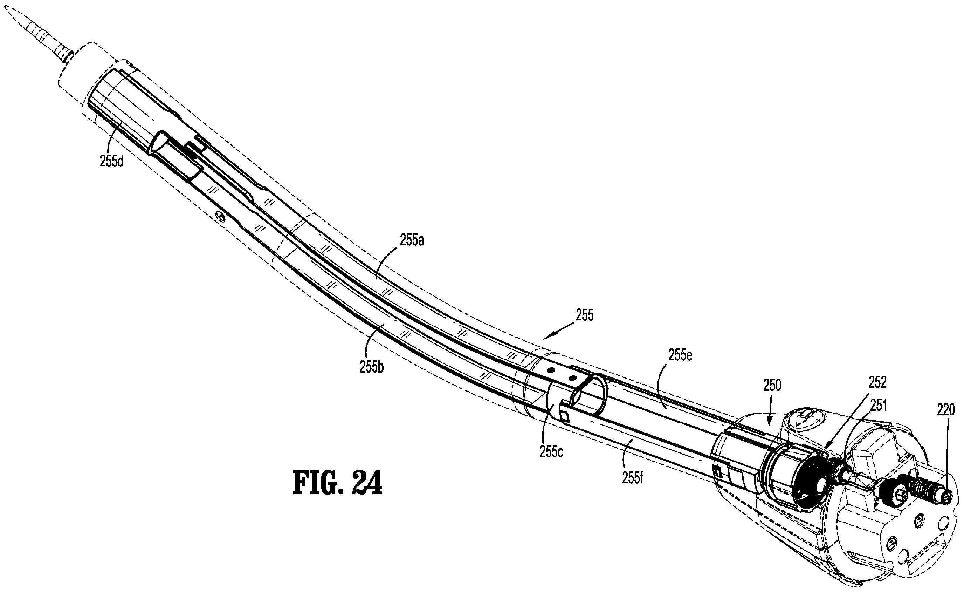

As shown in FIGS. 6 and 24, the second force/rotation transmitting/converting assembly 250 of adapter assembly 200 includes the second proximal drive shaft 214, as described above, a first coupling shaft 251, a planetary gear set 252, a staple lead screw 253, and a staple driver 254. The second force/rotation transmitting/converting assembly 250 of the adapter assembly 200 also includes an outer flexible band assembly 255 secured to the staple driver 254. The outer flexible band assembly 255 includes first and second flexible bands 255a, 255b laterally spaced and connected at proximal ends thereof to a support ring 255c and at distal ends thereof to a proximal end of a support base 255d. The outer flexible band assembly 255 further includes first and second connection extensions 255e, 255f extending proximally from the support ring 255c that are configured to operably connect the outer flexible band assembly 255 to the staple driver 254. The second force/rotation transmitting/converting assembly 250 functions to fire staples "S" (FIG. 13) of the reload 400 for formation against the anvil assembly 510.

In operation, as the second rotatable proximal drive shaft 214 is rotated due to a rotation of the second connector sleeve 220, as a result of the rotation of the second coupling shaft 124c (FIG. 2) of the handle assembly 100, the first coupling shaft 251 is caused to be rotated, which in turn causes the planetary gear set 252 to rotate. Rotation of the planetary gear set 252 causes contemporaneous rotation of the staple lead screw 253. As the staple lead screw 253 is rotated, the staple driver 254 is caused to be axially translated, which in turn causes the outer flexible band assembly 255 to be axially translated. As the outer flexible band assembly 255 is axially translated, the support base 255d presses against a driver adapter of a staple driver assembly (not shown) of the reload 400 to distally advance a driver and fire staples from a staple cartridge (not shown) of the reload 400 and against anvil assembly 510 for formation of the staples in underlying tissue.

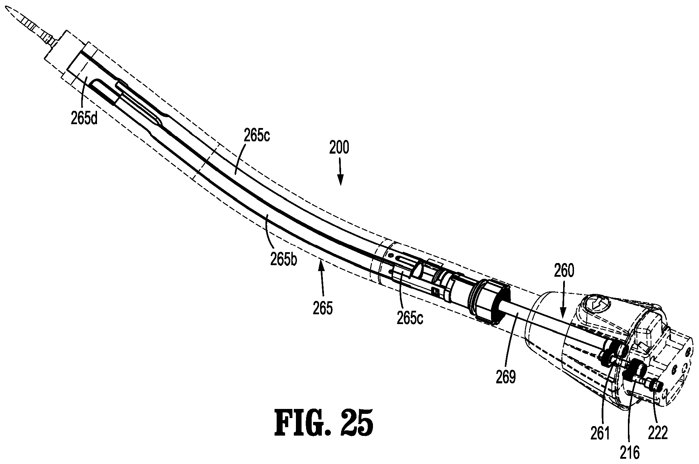

With reference to FIGS. 6 and 25, the third force/rotation transmitting/converting assembly 260 of the adapter assembly 200 includes the third proximal drive shaft 216, as described above, a second coupling shaft 261, a hollow shaft 269, a planetary gear set 262, a knife lead screw 263, and a knife driver 264. The third force/rotation transmitting/converting assembly 260 of adapter assembly 200 also includes an inner flexible band assembly 265 secured to the knife driver 264. The inner flexible band assembly 265 includes first and second flexible bands 265a, 265b laterally spaced and connected at proximal ends thereof to a support ring 265c and at distal ends thereof to a proximal end of a support base 265d. The third force/rotation transmitting/converting assembly 260 functions to fire an annular knife 444 (FIG. 13) of the reload 400.

In operation, as the third rotatable proximal drive shaft 216 is rotated due to a rotation of the third connector sleeve 222, as a result of the rotation of the third coupling shaft 124b (FIG. 2) of the handle assembly 100, the second coupling shaft 261 is caused to be rotated, which in turn causes the hollow shaft 269 to rotate. Rotation of the hollow shaft 269 results in contemporaneous rotation of the planetary gear set 262, which in turn causes the knife lead screw 263 to rotate. As the knife lead screw 263 is rotated, the knife driver 264 is caused to be axially translated, which in turn causes the inner flexible band assembly 265 to be axially translated. As the inner flexible band assembly 265 is axially translated, the support base 265d presses against a knife carrier (not shown) of the reload 400 to distally advance the knife carrier and fire the an annular knife (not shown) of the reload 400 against the anvil assembly 510 for cutting of tissue clamped in the reload 400.

Persons skilled in the art will understand that the structures specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art. For example, the electrical assemblies of the present disclosure may be configured for use with a plurality of different reloads via a plurality of respective adapter assemblies that are each configured for actuation and manipulation by a powered handle assembly and/or a robotic surgical system.

## Claims

1. An adapter assembly (200) for connecting a surgical stapler to an electromechanical handle assembly (100), the adapter assembly comprising:
an outer tube (206);
a trocar assembly (270) releasably securable within a distal end of the outer tube, the trocar assembly including a tubular trocar housing (272) defining a pair of openings or recesses in the outer surface therein (272a), wherein the pair of openings or recesses are in opposed radially extending relation to one another; and
a trocar assembly release mechanism (1275) configured to releasably secure the trocar assembly within the outer tube, the release mechanism including:
a pair of release buttons (1275a) rotatably supported on opposed radial sides of the outer tube (206), wherein each release button is selectively receivable within a respective opening of the pair of openings or recesses formed in the trocar housing, each release button being movable between a release condition and an engaged condition; and
a pair of biasing members (1275d) supported in the outer tube and associated with a respective one of the pair of release buttons, wherein the biasing members urge the pair of release buttons to the engaged condition and into engagement with the trocar housing;
wherein, when the release buttons are in the engaged, locked condition, the release buttons are disposed within the pair of openings or recesses of the trocar housing to maintain the trocar assembly connected to the outer tube; and
wherein, when the release buttons are in the release depressed, unlocked condition, the release buttons are disengaged from the pair of openings or recesses of the trocar housing to release the trocar assembly from the outer tube;
each release button defines a proximal face projecting to a tail (1275a"), wherein when the trocar assembly is properly inserted into the outer tube, the release buttons are urged radially inward, to the locked condition, by the biasing members (1275) rotating the release buttons such that the tails thereof are received in the openings or recesses in the trocar housing to provide a visual indication to an end user that the trocar assembly is properly inserted into the outer tube and wherein when the trocar assembly is not properly inserted into the outer tube, the release buttons are urged radially outward, to the unlocked condition, to provide a visual indication to an end user that the trocar assembly is not properly inserted into the outer tube.

2. The adapter assembly of claim 1, wherein the trocar release mechanism (1275) includes a pair of pivot pins (1275e), each pivot pin pivotably supporting a respective release button (1275a).

3. The adapter assembly of claim 2, wherein each release button (1275a) is substantially semi-circular, extending substantially 180° about a respective pivot pin thereof.

4. The adapter assembly of claim 3, wherein each release button (1275a) defines a distal face (1275a') against which a portion of a respective biasing member (1275d) engages to urge release button to the engaged condition and into engagement with the trocar housing.

5. The adapter assembly of any one of claims 1 to 4, wherein each release button (1275a) includes gripping features formed thereon.

## Patentansprüche

1. Adapteranordnung (200) zum Verbinden eines chirurgischen Klammergeräts mit einer elektromechanischen Griffanordnung (100), die Adapteranordnung umfassend:
ein Außenrohr (206);
eine Trokaranordnung (270), die lösbar innerhalb eines distalen Endes des Außenrohrs befestigbar ist, wobei die Trokaranordnung ein rohrförmiges Trokargehäuse (272) einschließt, das ein Paar von Öffnungen oder Aussparungen in der Außenfläche darin (272a) definiert, wobei das Paar von Öffnungen oder Aussparungen in entgegengesetzter, sich radial erstreckender Beziehung zueinander steht; und
einen Trokaranordnungsfreigabemechanismus (1275), der so konfiguriert ist, dass er die Trokaranordnung lösbar in dem Außenrohr sichert, wobei der Freigabemechanismus Folgendes umfasst:
ein Paar von Freigabeknöpfen (1275a), die drehbar auf gegenüberliegenden radialen Seiten des Außenrohrs (206) gelagert sind, wobei jeder Freigabeknopf selektiv in einer jeweiligen Öffnung des Paars von Öffnungen oder Aussparungen aufgenommen werden kann, die im Trokargehäuse ausgebildet sind, und jeder Freigabeknopf zwischen einem Freigabezustand und einem Eingriffszustand beweglich ist; und
ein Paar von Vorspannelementen (1275d), die in dem Außenrohr gelagert sind und mit jeweils einem der beiden Freigabeknöpfe verbunden sind, wobei die Vorspannelemente das Paar von Freigabeknöpfen in den Eingriffszustand und in Eingriff mit dem Trokargehäuse drängen; wobei die Freigabeknöpfe, wenn sie sich in dem eingerasteten, verriegelten Zustand befinden, innerhalb des Paars von Öffnungen oder Aussparungen des Trokargehäuses angeordnet sind, um die Trokaranordnung mit dem Außenrohr verbunden zu halten; und
wobei, wenn sich die Freigabeknöpfe in dem gedrückten, entriegelten Zustand befinden, die Freigabeknöpfe von dem Paar von Öffnungen oder Aussparungen des Trokargehäuses gelöst sind, um die Trokaranordnung von dem Außenrohr zu lösen;
jeder Freigabeknopf eine proximale Fläche definiert, die zu einem Ende (1275a") vorsteht, wobei die Freigabeknöpfe, wenn die Trokaranordnung ordnungsgemäß in das Außenrohr eingeführt ist, radial nach innen in den verriegelten Zustand gedrängt werden, durch die Vorspannelemente (1275), die die Freigabeknöpfe so drehen, dass ihre Enden in den Öffnungen oder Aussparungen im Trokargehäuse aufgenommen werden, um einem Endbenutzer eine visuelle Anzeige zu liefern, dass die Trokaranordnung ordnungsgemäß in das Außenrohr eingeführt ist, und wobei, wenn die Trokaranordnung nicht ordnungsgemäß in das Außenrohr eingeführt ist, die Freigabeknöpfe radial nach außen in den entriegelten Zustand gedrängt werden, um einem Endbenutzer eine visuelle Anzeige zu liefern, dass die Trokaranordnung nicht ordnungsgemäß in das Außenrohr eingeführt ist.

2. Adapteranordnung nach Anspruch 1, wobei der Trokarfreigabemechanismus (1275) ein Paar Drehstifte (1275e) einschließt, wobei jeder Drehstift schwenkbar einen jeweiligen Freigabeknopf (1275a) trägt.

3. Adapteranordnung nach Anspruch 2, wobei jeder Freigabeknopf (1275a) im Wesentlichen halbkreisförmig ist und sich im Wesentlichen 180° um einen entsprechenden Drehstift davon erstreckt.

4. Adapteranordnung nach Anspruch 3, wobei jeder Freigabeknopf (1275a) eine distale Fläche (1275a') definiert, an der ein Abschnitt eines jeweiligen Vorspannelements (1275d) eingreift, um den Freigabeknopf in den Eingriffszustand und in Eingriff mit dem Trokargehäuse zu drängen.

5. Adapteranordnung nach einem der Ansprüche 1 bis 4, wobei jeder Entriegelungsknopf (1275a) daran ausgebildete Griffelemente einschließt.

## Revendications

1. Ensemble adaptateur (200) permettant de relier une agrafeuse chirurgicale à un ensemble poignée électromécanique (100), l'ensemble adaptateur comprenant :
un tube extérieur (206) ;
un ensemble trocart (270) pouvant être fixé de manière amovible à l'intérieur d'une extrémité distale du tube extérieur, l'ensemble trocart comportant un logement de trocart tubulaire (272) définissant une paire d'ouvertures ou d'évidements dans la surface extérieure (272a) de celui-ci, dans lequel la paire d'ouvertures ou d'évidements est dans une relation s'étendant radialement de manière opposée l'une par rapport à l'autre ; et
un mécanisme de déclenchement de l'ensemble trocart (1275) conçu pour fixer de manière amovible l'ensemble trocart à l'intérieur du tube extérieur, le mécanisme de déclenchement comportant :
une paire de boutons de déclenchement (1275a) supportés de manière rotative sur les côtés radiaux opposés du tube extérieur (206), dans lequel chaque bouton de déclenchement est sélectivement recevable à l'intérieur d'une ouverture respective de la paire d'ouvertures ou d'évidements formés dans le logement de trocart, chaque bouton de déclenchement étant mobile entre un état de déclenchement et un état enclenché ; et
une paire d'éléments de sollicitation (1275d) supportés dans le tube extérieur et associés à un bouton respectif de la paire de boutons de déclenchement, dans lequel les éléments de sollicitation poussent la paire de boutons de déclenchement à l'état enclenché et en prise avec le logement de trocart ; dans lequel, lorsque les boutons de déclenchement sont dans l'état enclenché, verrouillé, les boutons de déclenchement sont disposés à l'intérieur de la paire d'ouvertures ou d'évidements du logement de trocart afin de maintenir l'ensemble trocart relié au tube extérieur ; et
dans lequel, lorsque les boutons de déclenchement sont dans l'état de déclenchement, déverrouillé, les boutons de déclenchement sont désolidarisés de la paire d'ouvertures ou d'évidements du logement de trocart pour libérer l'ensemble trocart du tube extérieur ;
chaque bouton de déclenchement définit une face proximale faisant saillie vers une queue (1275a"), dans lequel lorsque l'ensemble trocart est correctement inséré dans le tube extérieur, les boutons de déclenchement sont poussés radialement vers l'intérieur, à l'état verrouillé, par les éléments de sollicitation (1275) qui font tourner les boutons de déclenchement de telle sorte que les queues de ceux-ci sont reçues dans les ouvertures ou les évidements du logement de trocart afin de fournir une indication visuelle à un utilisateur final que l'ensemble trocart est correctement inséré dans le tube extérieur et dans lequel, lorsque l'ensemble trocart n'est pas correctement inséré dans le tube extérieur, les boutons de déclenchement sont poussés radialement vers l'extérieur, à l'état déverrouillé, afin de fournir une indication visuelle à un utilisateur final que l'ensemble trocart n'est pas correctement inséré dans le tube extérieur.

2. Ensemble adaptateur selon la revendication 1, dans lequel le mécanisme de déclenchement de trocart (1275) comporte une paire d'axes de pivotement (1275e), chaque axe de pivotement supportant de manière pivotante un bouton de déclenchement respectif (1275a).

3. Ensemble adaptateur selon la revendication 2, dans lequel chaque bouton de déclenchement (1275a) est sensiblement semi-circulaire, s'étendant sensiblement à 180° autour d'un axe de pivotement respectif de celui-ci.

4. Ensemble adaptateur selon la revendication 3, dans lequel chaque bouton de déclenchement (1275a) définit une face distale (1275a') contre laquelle une partie d'un élément de sollicitation respectif (1275d) vient en prise pour pousser le bouton de déclenchement à l'état enclenché et en prise avec le logement de trocart.

5. Ensemble adaptateur selon l'une quelconque des revendications 1 à 4, dans lequel chaque bouton de déclenchement (1275a) comporte des caractéristiques de préhension formées sur celui-ci.
